# EUROPÄISCHE PATENTSCHRIFT

(11) **EP 0 755 939 B1**
(45) Veröffentlichungstag und Bekanntmachung des Hinweises auf die Patenterteilung: **13.11.2002**
(21) Anmeldenummer: 96107500.9
(22) Anmeldetag: 10.05.1996
(51) Int. Cl.: C07H 5/06, C07H 15/04

(54) **Verfahren zur gezielten Synthese von Beta-glycosidisch verknüpften N-Acetylglucosaminderivaten**
Method for the targetted synthesis of beta-glycoside bound N-acetyl glucosamine derivatives
Méthode pour la synthèse ciblée des dérivés de N-acetylglucosamine liés à bêta-glycoside

(30) Priorität: 16.05.1995 DE 19517889
(43) Veröffentlichungstag der Anmeldung: 29.01.1997
(73) Patentinhaber: Aventis Research & Technologies GmbH & Co. KG, 65926 Frankfurt am Main (DE)
(72) Erfinder: Schmidt, Wolfgang, Dr., 65929 Frankfurt (DE); Kretzschmar, Gerhard, Dr., 65760 Eschborn (DE)
(74) Vertreter: Ackermann, Joachim, Dr.

(56) Entgegenhaltungen:
- EP-A- 0 489 162
- E.R. KUMAR ET AL.: "Preparation of ether-linked 2-acetamido-2-deoxy-beta glycolipids via zinc chloride promoted coupling of Ac4GlcNAc-Cl with lipid hydroxy groups" TETRAHEDR. LETT., Bd. 35, 1994, Seiten 505-8, XP002075304

## Beschreibung

Die Erfindung betrifft ein Verfahren zur gezielten Synthese von β-glycosidisch verknüpften N-Acetylglucosaminderivaten und im besonderen von N-Acetyl-2-amino-2-desoxy-β-D-glucopyranosiden.

Die Bedeutung der Kohlenhydrate im Rahmen physiologisch relevanter Erkennungsprozesse ist erst in den letzen Jahren näher untersucht und entschlüsselt worden (T. Feizi, Biochem. J. 1987, (245), 1; S. Hakamori, Adv. Cancer Res. 1989, (52), 257; M.P. Bevilacqua et. al., Science 1989, (243), 1160).

Man erkannte, daß die Kohlenhydrate, neben den Proteinen und Nucleinsäuren, der dritte wichtige Informationsträger des Lebens sind, da sie in der Regel eine große Anzahl von Stereozentren besitzen und so eine Vielzahl von Informationen beinhalten können. Ihre Anordnung auf der Zelloberfläche in Form von Liganden ermöglicht Ihnen, aufgrund von Rezeptor-Ligandenbindungen eine entscheidende Rolle bei der interzellulären Kommunikation und damit auch bei interzellulären Erkennungsprozessen zu spielen. Infolge dieser besonderen Bedeutung der Kohlenhydrate bei physiologisch relevanten Erkennungsprozessen kommt ihrer Synthese ein gesteigertes Interesse zu.

Ein wichtiger Baustein in der Oligosaccharidsynthese stellt das N-Acetyglucosamin (N-Acetyl-2-amino-2-desoxy-D-glucopyranose) 1 dar, das in in vielen physiologisch relevanten Oligosaccharidstrukturen vorkommt (z. B. J.C. Paulson et al., Science 1990, (250), 1130).

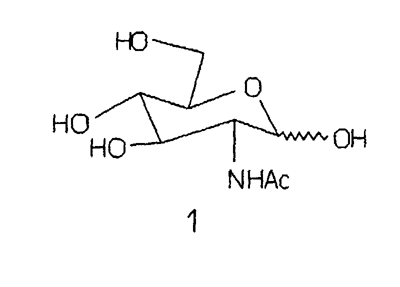

Bei der Synthese von Oligosacchariden werden Monosaccharidbausteine zusammengefügt, die in der Regel 4 bis 5 Hydroxylgruppen enthalten, welche sich in bezug auf deren Reaktivität nur wenig unterscheiden. Die gezielte Synthese der Oligosaccharide erfordert daher eine komplexe, aufeinander abgestimmte Schutzgruppenstrategie, die eine selektive Einführung und Abspaltung der verschiedenen Schutzgruppen ermöglicht. Bei der Glycosidsynthese ist es wichtig, die C-1-Position des Glycosyldonors zu aktivieren und dabei gleichzeitig die übrigen Hydroxylgruppen zu schützen, so daß diese nicht an der Kupplungsreaktion teilnehmen. Glycosyldonor und Glycosylakzeptor werden in der Regel in Gegenwart eines aktivierenden Katalysators miteinander verknüpft. Hierbei stellt die Kontrolle der Selektivität der glycosidischen Verknüpfung (d. h. α- oder β-Konfiguration des an der erzeugten glycosidischen Bindung beteiligten anomeren Kohlenstoffatoms) ein weiteres Problem dar. Zur Lösung dieses Problems sind die verschiedensten Verfahren entwickelt worden (Übersichten s. z. B. R. R. Schmidt, Pure & Appl. Chem. 1989, (61), 1257).

Viele Verfahren haben jedoch den Nachteil, daß sie unter Verwendung von teuren oder sehr giftigen Schwermetallen als Katalysatoren geringe Ausbeuten oder schlechte Selektivitäten ergeben.

Zur Darstellung von N-Acetylglucosaminderivaten sind eine Reihe verschiedenster N-Acetylglucosamindonoren eingesetzt worden (z. B. T. Mukaiyama et al., Chem. Lett. 1984, 907; K. Higashi, Chem. Pharm. Bull. 1990, (38), 3280).

Für eine effiziente Synthese in größerem Maßstab ist die gute Zugänglichkeit der Startmaterialien von besonderer Bedeutung. Von den verschiedenen N-Acetylglucosamindonoren ist das peracetylierte Chlorid der N-Acetyl-2-amino-2-desoxy-D-glucopyranose, das N-Acetyl-2-amino-2-desoxy-3,4,6-tri-O-acetyl-α-D-glucopyranosylchlorid 2, aus N-Acetyl-Glucosamin durch Umsetzung mit Acetylchlorid direkt in einer Stufe zugänglich (D. Horton, Org. Synth., Coll. Vol. V, 1973, 1).

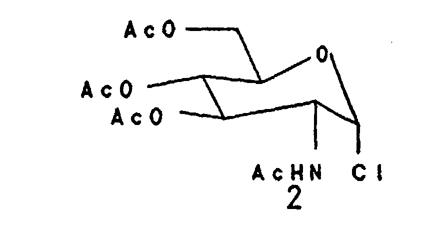

Als geeigneter Katalysator hat sich Zinkchlorid bewährt, mittels welchem bei der Umsetzung von N-Acetyl-2-amino-2-desoxy-3,4,6-tri-O-acetyl-α-Dglucopyranosylchlorid (2) mit Lactosederivaten α/β Verhältnisse bezüglich der Konfiguration der Produkte von bis zu 1/5 erreicht werden können (T. Norberg, J. Carbohydr. Chem. 1990, (9), 721).

In einem kürzlich beschriebenen Verfahren wurde der Glycosyldonor 2 mit verschiedenen Glycosylakzeptoren unter Zinkchlorid-Katalyse in Gegenwart von verschiedenen Cokatalysatoren umgesetzt (R. Bittman, Tetrahedron Lett., 1994, (35), 505). Als Cokatalysator wurde beispielsweise Tritylchlorid (Triphenylmethylchlorid) eingesetzt. Bei Verwendung dieser Katalysatorkombination waren jedoch längere Reaktionszeiten notwendig, und es traten Anomerisierungseffekte auf Kosten der β-Selektivität auf. Darüber hinaus reagierte eine Vielzahl verschiedener Glycosylakzeptoren unter diesen Bedingungen nicht mit dem Glycosyldonor 2.

Aufgabe der Erfindung ist es, ein Verfahren zur Synthese von β-glycosidisch verknüpften N-Acetylglucosaminderivaten, im besonderen von N-Acetyl-2-amino-2-desoxy-β-D-glucopyranosiden, bereitzustellen, welches bessere Ausbeuten sowie eine höhere β-Selektivität ergibt, keine Anomerisierung der erzeugten glycosidischen Bindung zur Folge hat und auf eine große Zahl von Glycosylakzeptoren, insbesondere Alkoholen, anwendbar ist.

Die Aufgabe wird erfindungsgemäß gelöst durch ein Verfahren zur Herstellung von N-Acetyl-2-amino-2-desoxy-β-D-glucopyranosiden durch Umsetzung von N-Acetyl-2-amino-2-desoxy-3,4,6-tri-O-acetyl-α-D-glucopyranosylchlorid (Donor 2) mit einem Glycosylakzeptor in Gegenwart von Zinkchlorid und eines Cokatalysators in einem Lösungsmittel, welches Verfahren sich dadurch auszeichnet, daß als Cokatalysator ein 4,4'-Dimethoxytriphenylmethylhalogenid eingesetzt wird.

Vorzugsweise werden bei dem erfindungsgemäßen Verfahren das Zinkchlorid und das 4,4'-Dimethoxytriphenylmethylhalogenid in einem molaren Verhältnis zueinander von 1:1 eingesetzt.

Als Lösungsmittel eignet sich insbesondere Dichlormethan.

Bei dem erfindungsgemäßen Verfahren wird vorzugsweise das wohlfeile 4,4'-Dimethoxytriphenylmethylchlorid als Cokatalysator eingesetzt.

Bei der Variation des Halogenids (Cl, Br, I) der Dimethoxytrityhalogenide (Darstellung siehe: Roedig, A. in Houben Weyl, Bd. V/4, 1960, 595 ff.) kann jedoch kein Unterschied in bezug auf Reaktivität und β-Selektivität beobachtet werden.

Der Einsatz der erfindungsgemäßen Katalysatorkombination führt zu einer deutlichen Verkürzung der Reaktionszeit im Vergleich zu dem bekannten Verfahren auch bei Einsatz im großen Maßstab.

Durch die veränderten Bedingungen kann ferner der Überschuß des Donors 2 gesenkt werden, und sehr unterschiedliche Alkohole können nahezu ohne Einschränkung als Kupplungspartner umgesetzt werden. Die chromatographische Reinigung der Produkte ist durch die veränderten Bedingungen ebenfalls deutlich verbessert. Infolge der kürzeren Reaktionszeit werden Anomerisierungseffekte weitestgehend vermieden, welches die gezielte Synthese von β-glycosidisch verknüpften N-Acetylglucosaminderivaten in guten Ausbeuten ermöglicht.

In den nachfolgenden Beispielen werden die Umsetzungen des N-Acetyl-2-amino-2-desoxy-3,4,6-tri-O-acetyl-α-D-glucopyranosylchlorids 2 mit jeweils unterschiedlichen Glycosylakzeptoren gemäß nachfolgendem Reaktionsschema beschrieben. Die dabei erhaltenen Verbindungen 3a-d sind wertvolle Zwischenstufen beim Aufbau komplexer Oligosaccharide (z.B. W.Stahl et al., Angew. Chem Int. Ed. Engl., 1994, (33), 2096).

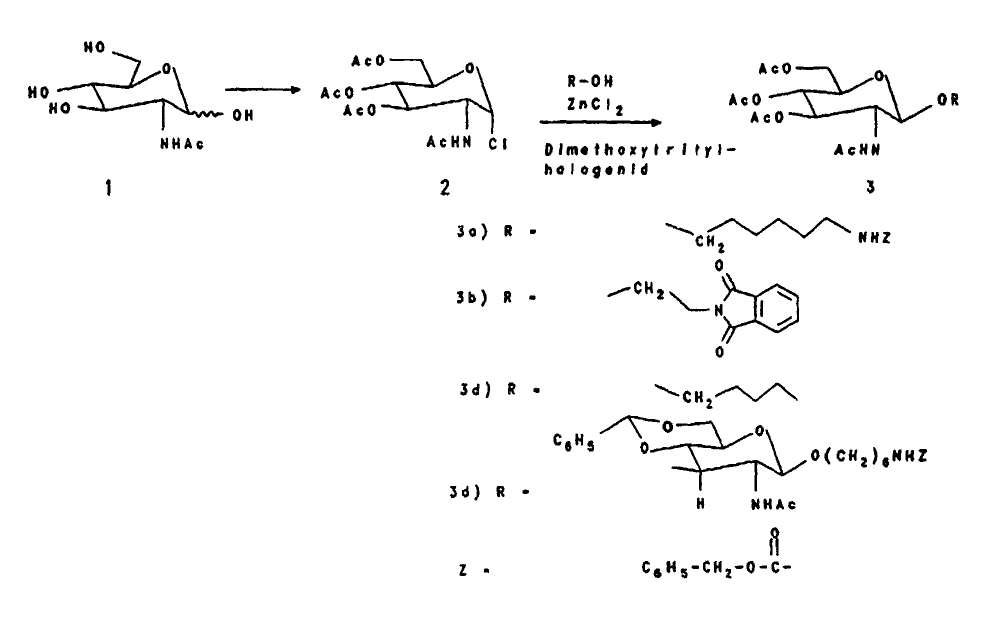

### Beispiele:

Die ¹H-NMR Spektren wurden mit dem Gerät WT 300 der Fa. Bruker bei 300 MHz aufgenommen. Zur Dünnschichtchromatographie wurden DC-Fertigplatten der Fa. Merck mit Kieselgel 60 F254 eingesetzt. Zur Säulenchromatograpie wurde Kieselgel 60 (Korngröße 0.040 - 0.063 mm, 230 - 400 mesh) der Fa. Merck verwandt.

### Beispiel 1

### Darstellung von N-Benzyloxycarbonyl-6-aminohexyl-N-acetyl-2-amino-2-desoxy-3,4,6-tri-O-acetyl-β-D-glucopyranosid 3a

Zu einer Suspension von Zink(II)-chlorid (1 eq., 80 g, 0,595 mol) und 4,4'-Dimethoxytritylchlorid (1 eq., 205 g, 0,595 mol) in trockenem Dichlormethan (3 l) werden das Chlorid 2 (1.3 eq., 282 g, 0,773 mol) und Z-6-Aminohexanol (1 eq., 150 g, 0,595 mol) gegeben. Das Gemisch wird 3 h bei Raumtemperatur gerührt (DC Kontrolle: Methylenchlorid/Methanol /Methanol 20/1). Nach Zugabe von Methylenchlorid (1 l) wird mit ges. Natriumhydrogencarbonatlösung gewaschen. Nach Entfernen des Lösungsmittels am Rotationsverdampfer wird der Rückstand säulenchromatographisch (Methylenchlorid/Methanol 100/1-20/1) gereinigt. Man erhält das Glycosid 3a in 93 % Ausbeute (321 g).
R_{f}-Wert:
0.35 ( Methylenchlorid/Methanol /Methanol 20 /1)
¹H-NMR (300 MHZ, CDCl₃):
7,38-7,28 (m, 5H); 6,00 (d, 1H); 5,29 (dd, 1H); 5,12 (dd, 1H); 5.05 (dd, 1H);
4.90 (t, 1H); 4.63 (d, J = 8,0 Hz, 1H); 4.25 (dd, 1H); 4,11 (dd, 1H);
3,90-3,72 (m, 2H); 3,67-3,58 (m, 1H); 3,52-3,43 (m, 1H); 3,26-3,08 (m, 2H);
2,12-1,94 (4 s, 12 H); 160-1,43 (m, 4 H); 1,39-1,28 (m, 4 H) ppm.

### Beispiel 2

### Darstellung von N-Phthaloylamido-2-aminoethyl-N-acetyl-2-amino-2-desoxy-3,4,6-tri-O-acetyl-β-D-glucopyranosid 3b

Zu einer Suspension von Zink(II)-chlorid (1 eq., 1,39 g) und 4,4'-Dimethoxytritylchlorid (1 eq., 1,22 g) in trockenem Dichlormethan (40 ml) werden das Chlorid 2 (1.3 eq., 4,97 g) und N-Hydroxyethylphthalimid (1 eq., 2,00 g) gegeben. Das Gemisch wird 1 h bei Raumtemperatur gerührt (DC Kontrolle: Methylenchlorid/Methanol 20/1). Nach Zugabe von Methylenchlorid wird mit ges. Natriumhydrogencarbonatlösung gewaschen. Nach Entfernen des Lösungsmittels am Rotationsverdampfer wird der Rückstand säulenchromatographisch (Methylenchlorid/Methanol /Methanol 100/1-40/1) gereinigt. Man erhält das Glycosid 3b in 91 % Ausbeute (4,94 g).
R_{f}-Wert: 0.35 ( Methylenchlorid/Methanol 20 /1)
¹H-NMR (300 MHZ, CDCl₃):
7,9 (m, 2 H); 7,7 (m, 2H); 5,95 (d, 1H); 5,30-5,05 (m, 2H); 4,95 (d, J = 8,0 Hz, 1H); 4,20-3,890 (m, 7H); 3,60 (dd, 1H), 2,10-2,00 (4 s, 12 H) ppm.

### Beispiel 3

### Darstellung von Pentyl-N-acetyl-2-amino-2-desoxy-3,4,6-tri-O-acetyl-β-Dglucopyranosid 3c

Zu einer Suspension von Zink(II)-chlorid (1 eq., 1,55 g) und 4,4'-Dimethoxytritylchlorid (1 eq., 3,84 g) in trockenem Dichlormethan (40 ml) werden das Chlorid 2 (1.3 eq., 5,38 g) und n-Pentanol (1 eq., 2,00 g) gegeben. Das Gemisch wird 1 h bei Raumtemperatur gerührt (DC Kontrolle:
Methylenchlorid/Methanol 20/1). Nach Zugabe von Methylenchlorid wird mit ges. Natriumhydrogencarbonatlösung gewaschen. Nach Entfernen des Lösungsmittels am Rotationsverdampfer wird der Rückstand säulenchromatographisch (Methylenchlorid/ Methanol 100/1-40/1) gereinigt. Man erhält das Glycosid 3c in 85 % Ausbeute (4,01 g).
R_{f}-Wert: 0.35 ( Methylenchlorid/Methanol /Methanol 20 /1)
¹H-NMR (300 MHZ, CDCl₃):
7,38-7,28 (m, 5H); 6,00 (d, 1H); 5,30 (dd, 1H); 5,15 (dd, 1H); 5.05 (dd, 1H);
4.95 (t, 1H); 4.65 (d, J = 8,5 Hz, 1H); 4.20 (dd, 1H); 4,10 (dd, 1H);
3,90-3,70 (m, 2H); 3,67-3,58 (m, 1H); 3,52-3,43 (m, 1H); 2,12-1,94 (4 s, 12 H); 165-1,30 (m, 8 H) ppm.

### Beispiel 4

### Darstellung von N-Benzyloxycarbonyl-6-aminohexyl-N-acetyl-2-amino-2-desoxy-3-O-(N-acetyl-2-amino-2-desoxy-3,4,6-tri-O-acetyl-β-D-glucopyranosyl)-4,6-Obenzyliden-β-D-glucopyranosid 3d

Zu einer Suspension von Zink(II)-chlorid (1 eq., 0.49 g) und 4,4'-Dimethoxytritylchlorid (1 eq., 1,23 g) in trockenem Dichlormethan (40 ml) werden das Chlorid 2 (1.3 eq., 1,72 g) und N-Benzyloxycarbonyl-6-aminohexyl-N-acetyl-2-amino-2-desoxy-4,6-O-benzyliden-ß-D-glucopyranosid (1 eq., 2,00 g ) gegeben. Das Gemisch wird 1 h bei Raumtemperatur gerührt (DC Kontrolle:
Methylenchlorid/Methanol 20/1). Nach Zugabe von Methylenchlorid wird mit ges. Natriumhydrogencarbonatlösung gewaschen. Nach Entfernen des Lösungsmittels am Rotationsverdampfer wird der Rückstand säulenchromatographisch (Methylenchlorid/ Methanol 100/1-40/1) gereinigt. Man erhält das Glycosid 3d in 86 % Ausbeute (2,74 g).
R_{f}-Wert: 0.35 ( Methylenchlorid/Methanol /Methanol 20 /1)
¹H-NMR (300 MHZ, CDCl₃):
7,50-7,25 (m, 10H); 5,80 (m; 1H); 5,55 (s, 1H); 5,18 (m, 1H); 5,05 (s, 2H);
4,75 (d, 1H); 4,69 (d, J = 8,0 Hz, 1H); 4,60 (d, J = 8.0 Hz, 1H); 4.32-4.00 Im, 5H); 3,80 (m, 3H); 3,50 (m, 1H); 3,17 (m, 2H); 2,15-1,90 (5 s, 15H);
1,60-1,35 (m, 8H) ppm.

## Patentansprüche

1. Verfahren zur Herstellung von N-Acetyl-2-amino-2-desoxy-β-Dglucopyranosiden durch Umsetzung von N-Acetyl-2-amino-2-desoxy-3,4,6-tri-O-acetyl-α-D-glucopyranosylchlorid mit einem Glycosylakzeptor in Gegenwart von Zinkchlorid und eines Cokatalysators in einem Lösungsmittel, **dadurch gekennzeichnet, daß** als Cokatalysator ein 4,4'-Dimethoxytriphenylmethylhalogenid eingesetzt wird.

2. Verfahren nach Anspruch 1, **dadurch gekennzeichnet, daß** das Zinkchlorid und das 4,4'-Dimethoxytriphenylmethylhalogenid in einem molaren Verhältnis zueinander von 1:1 eingesetzt werden.

3. Verfahren nach Anspruch 1 oder 2, **dadurch gekennzeichnet, daß** als Lösungsmittel Dichlormethan verwendet wird.

4. Verfahren nach einem der Ansprüche 1 bis 3, **dadurch gekennzeichnet, daß** als Cokatalysator 4,4'-Dimethoxytriphenylmethylchlorid eingesetzt wird.

## Claims

1. A process for the preparation of N-acetyl-2-amino-2-deoxy-β-D-glucopyranosides by reaction of N-acetyl-2-amino-2-deoxy-3,4,6-tri-O-acetyl-α-D-glucopyranosyl chloride with a glycosyl acceptor in the presence of zinc chloride and of a cocatalyst in a solvent, which comprises employing as cocatalyst a 4,4'-dimethoxytriphenylmethyl halide.

2. The process as claimed in claim 1, wherein the zinc chloride and the 4,4'-dimethoxytriphenylmethyl halide are employed in a molar ratio to one another of 1:1.

3. The process as claimed in claim 1 or 2, wherein the solvent used is dichloromethane.

4. The process as claimed in one of claims 1 to 3, which comprises employing as cocatalyst 4,4'-dimethoxytriphenylmethyl chloride.

## Revendications

1. Procédé pour la fabrication de N-acétyl-2-amino-2-désoxy-β-D-glucopyranosides par réaction de chlorure de N-acétyl-2-amino-2-désoxy-3,4,6-tri-O-acétyl-α-D-glucopyranosyle avec un accepteur de glycosyle en présence de chlorure de zinc et d'un co-catalyseur dans un solvant, **caractérisé en ce qu'**on utilise comme co-catalyseur un halogénure de 4,4'-diméthoxytriphénylméthyle.

2. Procédé selon la revendication 1, **caractérisé en ce que** le chlorure de zinc et l'halogénure de 4,4'-diméthoxytriphénylméthyle sont mis en oeuvre dans un rapport molaire l'un par rapport à l'autre de 1 : 1.

3. Procédé selon la revendication 1 ou 2, **caractérisé en ce qu'**on utilise comme solvant le dichlorométhane.

4. Procédé selon une des revendications 1 à 3, **caractérisé en ce qu'**on utilise comme co-catalyseur le chlorure de 4,4'-diméthyoxytriphénylméthyle.
